# EUROPÄISCHE PATENTSCHRIFT

(11) **EP 4 065 555 B1**
(45) Veröffentlichungstag und Bekanntmachung des Hinweises auf die Patenterteilung: **05.08.2026**
(21) Anmeldenummer: 20811398.5
(22) Anmeldetag: 27.11.2020
(51) Int. Cl.: C07C 319/14, C07C 321/14

(54) **VERBESSERTES VERFAHREN ZUR HERSTELLUNG VON UNSYMMETRISCHEN DIALKYLSULFIDEN**
IMPROVED METHOD FOR THE PREPARATION OF ASYMMETRICAL DIALKYL SULPHIDES
PROCÉDÉ AMÉLIORÉ DE FABRICATION DE SULFURES DE DIALKYLE NON SYMÉTRIQUES

(30) Priorität: 28.11.2019 EP 19212191
(43) Veröffentlichungstag der Anmeldung: 05.10.2022
(73) Patentinhaber: Syngenta Crop Protection AG, 4058 Basel (CH)
(72) Erfinder: GROSSMANN, Andre, 50735 Köln (DE)
(74) Vertreter: SYNGENTA IP
(86) Internationale Anmeldenummer: PCT/EP2020/083655
(87) Internationale Veröffentlichungsnummer: WO 2021/105376

(56) Entgegenhaltungen:
- CN-A- 108 752 279
- US-A1- 2011 306 797
- E.I. TROYANSKII ET AL: "Polar control in the remote oxidative functionalization of sulfones", BULLETIN OF THE ACADEMY OF SCIENCES OF THE USSR, DIVISION OF CHEMICAL SCIENCE (ENGLISH TRANSLATION), 1 January 1988 (1988-01-01), pages 1919 - 1925, XP055692360, Retrieved from the Internet <URL:https://link.springer.com/content/pdf/10.1007/BF00962514.pdf> [retrieved on 20200506]
- D. S. TARBELL ET AL: "The Isomerization by Base of Alkyl Allyl Sulfides to Alkyl Propenyl Sulfides. The Mechanism of the Reaction 1", JOURNAL OF THE AMERICAN CHEMICAL SOCIETY, vol. 78, no. 10, 1 May 1956 (1956-05-01), US, pages 2259 - 2264, XP055692331, ISSN: 0002-7863, DOI: 10.1021/ja01591a063

## Beschreibung

Die Erfindung betrifft ein verbessertes Verfahren zur Herstellung von unsymmetrischen Dialkylsulfiden durch Umsetzung eines Monoalkylsulfides zumindest mit einem Alkylhalogenid in Gegenwart von Base und in Abwesenheit von organischen Lösungsmitteln.

Solche unsymmetrischen Dialkylsulfide stellen Reagenzien dar, aus denen Sulfoniumsalze erzeugt werden können, die ihrerseits vorteilhaft in Epoxidierungsreaktionen, wie beispielsweise der Corey-Chaykovsky-Reaktion, eingesetzt werden können. Über diese Epoxidierungsreaktionen sind Zwischenstufen erhältlich, die in der Synthese von pharmazeutischen Wirkstoffen, beispielsweise Fluconazol, oder Pflanzenschutzwirkstoffen, beispielsweise Cyproconazol, eingesetzt werden können.

Zur Herstellung von unsymmetrischen Dialkylsulfiden sind bereits chemische Verfahren bekannt. Beispielsweise offenbart C. Kimura et al., Yuki Gosei Kagaku Kyokaishi (1977), 35(8), 669-71, die Herstellung von Ethyloctylsulfid durch Umsetzung von Octanthiol mit Ethylbromid in Gegenwart eines Phasentransferkatalysators, beispielsweise Tridodecylethylammoniumbromid, und in Gegenwart von Natriumhydroxid. Hierbei werden die beiden Reaktanden Natronlauge und Phasentransferkatalysator zusammengegeben und unter starker Vermischung bei 0 bis 30 °C zur Reaktion gebracht.

E.I. Troyanskii et al., Bulletin of the Academy of Sciences of the USSR, 1988, pp 1919-1925, beschreibt ein Verfahren zur Herstellung von n-Butylmethylsulfid bei welchem n-Butylmercaptan mit Methyliodid in Gegenwart von Natriumhydroxid und einem Phasentransfer-Katalysator (Tetraoctylammoniumchlorid) umgesetzt wird. Das Verfahren wird jedoch in Anwesenheit des organischen Lösungsmittels Benzol durchgeführt.

US 2011/0306797 A1 beschreibt ein Verfahren zur Herstellung von unsymmetrischen Dialkylsulfiden in Abwesenheit von organischen Lösungsmitteln, in dem das Alkylsulfid mit einer niedrigeren oder gleichen Anzahl an Kohlenstoffatomen im 50 %igen molaren Überschuss gegenüber dem Alkylhalogenid mit der höheren oder gleichen Anzahl an Kohlenstoffatomen eingesetzt wird. Das dabei entstehende Dialkylsulfid wird nicht isoliert, sondern in eine organische Phase extrahiert, in der es nachfolgend zum Sulfon oxidiert wird. Bezogen auf das eingesetzte Alkylhalogenid beträgt die angegebene Ausbeute des Sulfons zwischen 85 und 88 Prozent der Theorie. Umgerechnet auf das eingesetzte Alkylsulfid beträgt die angegebene Ausbeute des Sulfons jedoch unter 60 Prozent der Theorie.

JP2010285408A offenbart ein Verfahren zur Herstellung von Dodecylmethylsulfid aus Dodecylthiol und Natriummethylthiolat in Gegenwart des Phasentransferkatalysators Tetrabutylammoniumbromid bei Temperaturen von 80 °C in Ausbeuten von mindestens 95%.

JP2010285378A und EP2441751 A1 offenbaren ein Verfahren zur Herstellung von Dialkylsulfiden, beispielsweise Ethylisopropylsulfid, aus Alkylhalogeniden, beispielsweise 2-Brompropan, und Alkylmercaptansalzen, beispielsweise Natriumethanthiolat, erhalten aus Ethanthiol und Natronlauge, in Gegenwart von Base, beispielsweise Natriumhydroxid, einem Reduktionsmittel, beispielsweise Natriumborhydrid, und Phasentransferkatalysator, beispielsweise Tetrabutylammoniumbromid bei Temperaturen von 0 bis 50 °C in Ausbeuten von mindestens 95 %. Hierbei dient das Reduktionsmittel zur Vermeidung der Bildung von Alkyldisulfiden.

EP2351735 A1 offenbart ein Verfahren zur Herstellung von Dialkylsulfiden, beispielsweise Butylisobutylsulfid, aus Alkylhalogeniden, beispielsweise 1-Brom-2-methylpropan und Alkylmercaptansalzen, beispielsweise Natriumbutanthiolat, erhalten aus Butanthiol und Natronlauge, in Gegenwart von Base, beispielsweise Natriumhydroxid, einem Reduktionsmittel, beispielsweise Natriumborhydrid, und Phasentransferkatalysator, beispielsweise Tetrabutylammoniumbromid bei Temperaturen von 0 bis 50 °C. Diese werden nicht isoliert, sondern direkt zu den entsprechenden Sulfonen oxidiert.

Diese Verfahren liefern zwar die gewünschten Produkte in sehr guten Ausbeuten, weisen jedoch bei deren Anwendung im größeren Maßstab, beispielsweise im 20 bis 100 Litermaßstab, entscheidende Nachteile auf. Werden beispielsweise, wie in C. Kimura et al., alle Reaktanden der Reaktion, also Alkylsulfid, Alkylhalogenid, Base und Phasentransferkatalysator, auf einmal bei Raumtemperatur oder bei erhöhter Temperatur, beispielweise 50 °C, auf einmal zusammengegeben, kann die dabei entstehende Reaktionswärme die Temperatur des Reaktionsgemisches leicht auf Temperaturen von über 100 °C bringen. Zusätzlich kann bei dieser Verfahrensführung durch einen hohen Überschuss an Base das Salz der Base und des Alkylsulfids in größerem Ausmaß entstehen und auskristallisieren. Beides kann im technischen Maßstab gegebenenfalls unkontrollierbar sein. Anderseits führt eine Reaktion eines Alkylhalogenids mit einem kommerziell erhältlichen oder vor der Umsetzung hergestellten Natriumalkylthiolat und/oder die Zugabe von weiteren Reaktanden wie beispielsweise Reduktionsmitteln, zu erhöhten Herstellungskosten und/oder zu einem erhöhten Verfahrensaufwand. Die Erhöhung der Herstellungskosten und/oder des Verfahrensaufwands resultiert auch aus dem Einsatz anderer Lösungsmittel außer Wasser, die gegebenenfalls nach der Reaktion als Abfall entsorgt werden müssen.

Deshalb bestand nach wie vor der Bedarf und somit die Aufgabe an einem günstigen, abfallarmen und einfachen Verfahren zur Herstellung von unsymmetrischen Dialkylsulfiden, das auch im industriellen Maßstab zum einen die hohen Ausbeuten der Verfahren des Standes der Technik liefert und zum anderen deren Nachteile nicht aufweist.

Es wurde nun ein verbessertes Verfahren zur Herstellung von unsymmetrischen Dialkylsulfiden der Formel (I),

R¹-S-R² (I),

gefunden, umfassend die Umsetzung eines Alkylsulfides der Formel (II),

   H-S-R² (II),
mit einem Alkylhalogenid der Formel (III),

   R¹-X (III),
worin in den Formeln (I), (II) und (III)
R¹ für einen C₁-C₃- Alkylrest, bevorzugt für Methyl,
R² für einen C₄-C₁₂ Alkylrest,
X für Halogen, bevorzugt für Chlorid, Bromid, Iodid, besonders bevorzugt für Chlorid, stehen, zumindest in Gegenwart einer Base, bevorzugt in Gegenwart eines Alkalimetallhydroxides,
wobei ein Reaktionsgemisch entsteht, und die Temperatur des Reaktionsgemisches während der Umsetzung in einem Bereich von 15 bis 100 °C, bevorzugt von 15 bis 50 °C, besonders bevorzugt von 25 bis 40 °C, liegt und worin das Alkylhalogenid der Formel (III) in einer Menge von 0,9 bis 3 Moläquivalenten bezogen auf das Alkylsulfid der Formel (II) eingesetzt wird und wobei die Umsetzung des Alkylhalogenids der Formel (III) und des Alkylsulfides der Formel (II) in Abwesenheit von organischen Lösungsmitteln erfolgt.

In dem erfindungsgemäßen Verfahren erfolgt die Umsetzung bevorzugt in Gegenwart von Wasser und einem Phasentransferkatalysator, bevorzugt einem quartären Ammoniumsalz der Formel (IV),

R³R⁴₃N⁺Y⁻ (IV),

worin
R³ für Wasserstoff oder für Methyl, bevorzugt für Methyl,
R⁴ für einen C₄-C₁₂ Alkylrest, bevorzugt für Octyl und/oder Decyl,
X für Halogen, bevorzugt für Chlorid, Bromid, oder Iodid, stehen.

Vorzugsweise steht R² in Formel (I) und Formel (II) für n-Butyl, n-Pentyl, n-Hexyl, n-Heptyl, n-Octyl, n-Nonyl, n-Decyl, n-Undecyl oder n-Dodecyl. Besonders bevorzugt steht R² in Formel (I) und Formel (II) für n-Butyl.

In dem erfindungsgemäßen Verfahren wird bevorzugt zu einer Mischung des Alkylsulfides der Formel (II) und dem Phasentransferkatalysator, bevorzugt dem quartären Ammoniumsalz der Formel (IV), und gegebenenfalls Wasser, gleichzeitig Base, bevorzugt das Alkalimetallhydroxid, und das Alkylhalogenid der Formel (III), gegeben.

Die Temperatur des Reaktionsgemisches wird vorzugsweise nicht durch externe Wärmezufuhr, sondern durch die Zugabegeschwindigkeit der Base, bevorzugt des Alkalimetallhydroxids, geregelt. Da die Reaktion der Base, bevorzugt des Alkalimetallhydroxids, mit dem Alkylsulfid der Formel (II), bei der das entsprechende Salz des Alkylsulfid der Formel (II) entsteht, exotherm ist, kann die Temperatur des Reaktionsgemisches durch die Geschwindigkeit, mit der die Base, bevorzugt das Alkalimetallhydroxid, zugegeben wird, einfach und sicher auf den erforderlichen Temperaturbereich von 15 bis 100 °C, bevorzugt von 15 bis 50 °C, besonders bevorzugt von 25 bis 40 °C, eingestellt und gehalten werden, und worin das Alkylhalogenid der Formel (III) in einer Menge von 0,9 bis 3 Moläquivalenten bezogen auf das Alkylsulfid der Formel (II) eingesetzt wird und wobei die Umsetzung des Alkylhalogenids der Formel (III) und des Alkylsulfides der Formel (II) in Abwesenheit von organischen Lösungsmitteln erfolgt..

Für das erfindungsgemäße Verfahren ist vorteilhaft, wenn zu Beginn der Umsetzung zu der Mischung aus Alkylsulfid der Formel (II), gegebenenfalls Phasentransferkatalysator, bevorzugt dem quartären Ammoniumsalz der Formel (IV), und gegebenenfalls Wasser, von 0,01 bis 0,05 Mol, bevorzugt 0,02 bis 0,04 Mol, Base, bevorzugt Alkalimetallhydroxid, bezogen auf 1,0 Mol Alkylsulfid der Formel (II) zugegeben werden, bevor Alkylhalogenid der Formel (III) zum Reaktionsgemisch zugegeben wird. Dadurch liegt bereits vom Beginn der Umsetzung an ein Anteil des Alkylsulfides der Formel (II) als Salz der Base, bevorzugt des Alkalimetallhydroxids, vor, das direkt mit zugegebenem Alkylhalogenid der Formel (III) reagieren kann.

Im weiteren Verlauf der Umsetzung wird Base, bevorzugt Alkalimetallhydroxid, dem Reaktionsgemisch zugegeben. Dabei ist für das das erfindungsgemäße Verfahren vorteilhaft, wenn gemeinsam mit der Base, bevorzugt dem Alkalimetallhydroxid, das Alkylhalogenid der Formel (III), besonders bevorzugt in gasförmigem Zustand, dem Reaktionsgemisch zugegeben wird. Besonders bevorzugt wird das Alkylhalogenid der Formel (III) gemeinsam mit der Base, bevorzugt mit dem Alkalimetallhydroxid, dem Reaktionsgemisch derart zugegeben, dass zu jedem Zeitpunkt der Umsetzung genügend Alkylhalogenid der Formel (III) im Reaktionssystem vorhanden ist, sodass die Umsetzung zum Dialkylsulfid der Formel (I) möglichst schnell erfolgt. In einer bevorzugten Ausführungsform wird dies dadurch erreicht, dass die Umsetzung in einem geschlossenen Reaktor erfolgt. Der Fachmann wird dabei die Grenzen der Dichtheit und der Druckbeständigkeit des Reaktors basierend auf seinem Fachwissen den jeweiligen Anforderungen anpassen. Vorzugsweise wird dabei in dem Reaktor enthaltend die Mischung aus Alkylsulfid der Formel (II), gegebenenfalls Phasentransferkatalysator, bevorzugt dem quartären Ammoniumsalz der Formel (IV), gegebenenfalls Wasser, sowie von 0,01 bis 0,05 Mol, bevorzugt 0,02 bis 0,04 Mol, Base, bevorzugt Alkalimetallhydroxid, bezogen auf 1,0 Mol Alkylsulfid der Formel (II) der Druck im Reaktor zu Beginn der Umsetzung auf von 500 bis 1500 hPa, eingestellt, und dann Alkylhalogenid der Formel (III) derart gasförmig zudosiert wird, bis der Druck im Reaktor um 100 bis 700 hPa, bevorzugt von 150 bis 300 hPa, gegenüber dem Anfangsdruck gestiegen ist. Damit liegt bevorzugt im Reaktor während der Umsetzung ein Partialdruck an Alkylhalogenid der Formel (III) - gemessen im gasförmigen Raum oberhalb des flüssigen Reaktionsgemisches innerhalb des Reaktors - von 100 bis 1500 hPa, bevorzugt von 100 bis 700 hPa, vor. Da sich das Alkylhalogenid der Formel (III) im Reaktionsgemisch löst und während der Reaktion verbraucht wird, würde der Druck im Reaktor - gemessen im gasförmigen Raum oberhalb des flüssigen Reaktionsgemisches innerhalb des Reaktors - ohne weitere Zugabe von Alkylhalogenid der Formel (III) sinken. Deshalb wird, nachdem die Zugabe von Base, bevorzugt von Alkalimetallhydroxid, begonnen hat, der Partialdruck an Alkylhalogenid der Formel (III) durch weitere Zudosierung von Alkylhalogenid der Formel (III) in einem Bereich von 100 bis 1500 hPa, bevorzugt von 100 bis 700 hPa, eingestellt. Mit der Regelung der Zugabe an Alkylhalogenid der Formel (III), die über die Einstellung des erhöhten Drucks im Reaktor geregelt wird, wird sichergestellt, dass zu jedem Zeitpunkt der Umsetzung genügend Alkylhalogenid der Formel (III) im Reaktionssystem vorhanden ist. In dieser bevorzugten Ausführungsform wird darüber hinaus über die Messung der zugegebenen Masse an Alkylhalogenid der Formel (III) der Zeitpunkt bestimmt, wann die erforderliche Masse des Alkylhalogenids der Formel (III) erreicht wurde und die Zugabe dann beendet wird.

Während der bevorzugten gemeinsamen Dosierung von Base, bevorzugt von Alkalimetallhydroxid, und Alkylhalogenid der Formel (III), wird jeweils nur ein Anteil des vorgelegten Alkylsulfids der Formel (II) in das entsprechende Salz der Base, bevorzugt des Alkalimetallhydroxids, umgewandelt und durch das ebenfalls zudosierte Alkylhalogenid der Formel (III) schnell zum Dialkylsulfid der Formel (I) umgesetzt. Durch diese bevorzugte Ausführungsform wird vermieden, dass sich während der Umsetzung größere Anteile an Salz der Base, bevorzugt des Alkalimetallhydroxids, und/oder des Alkylsulfides der Formel (II) bilden.

Weitere bevorzugte Ausführungsformen können in der diskontinuierlichen oder kontinuierlichen Zugabe der beiden Reaktanden a) Base, bevorzugt Alkalimetallhydroxid, und b) Alkylhalogenid der Formel (III) zu der Mischung aus Alkylsulfid der Formel (II) und gegebenenfalls Phasentransferkatalysator, bevorzugt dem quartären Ammoniumsalz der Formel (IV), und gegebenenfalls Wasser, bestehen. Erfindungsgemäß wird dabei "kontinuierliche Zugabe" derart definiert, dass sie ohne Unterbrechung erfolgt. "Diskontinuierliche Zugabe" bedeutet erfindungsgemäß, dass die Zugabe, beispielsweise in mehreren diskreten Portionen, mit Unterbrechungen erfolgt. Dabei können bei der diskontinuierlichen Zugabe sowohl zeitliche Phasen kontinuierlicher und ebenso zeitliche Phasen mit diskontinuierlicher Zugabe enthalten sein.

Im Folgenden wird das erfindungsgemäße Verfahren näher beschrieben.

Als Basen können beispielsweise Alkalimetallhydroxide, wie Natriumhydroxid oder Kaliumhydroxid, Alkalimetallcarbonate, wie Natriumcarbonat oder Kaliumcarbonat; Alkalimetallalkoholate, wie Natriumalkoholate oder Kaliumalkoholate eingesetzt werden. Bevorzugt wird im erfindungsgemäßen Verfahren als Base ein oder mehrere Alkalimetallhydroxide, besonders bevorzugt Natriumhydroxid oder Kaliumhydroxid, oder deren Mischungen, eingesetzt. Ganz besonders bevorzugt wird Natriumhydroxid als Base eingesetzt. Dies kann beispielsweise Natriumhydroxid in fester Form, bevorzugt als Schuppen oder Pulver, oder als Lösung sein.

Vorzugsweise beträgt die Menge der Base von 0,9 bis 2 Mol, bevorzugt von 1,0 bis 1,2 Mol, bezogen auf 1 Mol Alkylsulfid der Formel (II).

Bevorzugt beträgt die Menge des Alkylhalogenids der Formel (III) von 1,1 bis 1,5 Mol, bezogen auf 1 Mol des Alkylsulfid der Formel (II).

Das erfindungsgemäße Verfahren wird ohne zusätzliche organische Lösungsmittel, das bedeutet in Abwesenheit von organischen Lösungsmitteln, beispielsweise Ether, beispielsweise Tetrahydrofuran oder 1,4-Dioxan, Alkohole, beispielsweise Methanol oder Ethanol, Ketone, beispielsweise Aceton, Ester, beispielsweise Ethylacetat, Kohlenwasserstoffe, beispielsweise Cyclohexan, halogenierte Kohlenwasserstoffe, beispielsweise Dichlormethan oder Chlorbenzol, oder Nitrile, beispielsweise Acetonitril, durchgeführt. Im erfindungsgemäßen Verfahren kann dagegen bevorzugt Wasser als Lösungsmittel fungieren. Da sowohl die eingesetzten Edukte Alkylsulfid der Formel (II) und Alkyhalogenid der Formel (III) als auch das Produkt Dialkylsulfid der Formel (I) in Wasser nur begrenzt oder gar nicht löslich sind, findet die Reaktion zweiphasig statt. Daher wird das Reaktionsgemisch bevorzugt vor, während und nach Zugabe des Alkylhalogenids der Formel (III) mechanisch oder hydraulisch derart vermischt, dass die Umsetzung der Reaktanden innerhalb von weniger als 24 Stunden, bevorzugt innerhalb von weniger als 12 Stunden, zu mindestens 90 Prozent abgeschlossen ist. Eine zu geringe Durchmischung würde die Reaktionszeiten unnötig verlängern. Üblicherweise beträgt die Menge an Wasser, die dem Reaktionsgemisch als Wasser oder Lösung der Base, bevorzugt des Alkalimetallhydroxids, zugefügt wird, von 1 bis 20 kg, bevorzugt von 3 bis 15 kg, bezogen auf 1 kg Alkylsulfid der Formel (II).

Es hat sich im erfindungsgemäßen Verfahren die Verwendung von Phasentransferkatalysatoren als vorteilhaft herausgestellt. Phasentransferkatalysator im Sinne des erfindungsgemäßen Verfahrens sind beispielsweise quartäre Ammoniumsalze wie Benzyltriethylammoniumbromid, Benzyltrimethylammoniumbromid, Trihexadecylethylammoniumchlorid, Tridodecylmethylammoniumchlorid, Tridecylmethylammoniumchlorid, Trioctyltrimethylammoniumchlorid, Trioctyltrimethylammoniumbromid, Tetra-n-butylammoniumbromid, Tetraethylammoniumchlorid, oder Trioctylmethylammoniumbromid; sowie quartäre Phosphoniumsalze, wie Hexadodecyltriethylphosphoniumbromid, Hexadecyltributylphosphoniumchlorid, oder Tetra-n-butylphosphoniumchlorid. Bevorzugt wird quartäres Ammoniumsalz der Formel (IV),

R³R⁴₃N⁺Y⁻ (IV),

eingesetzt, worin
R³ für Methyl,
R⁴ für Octyl und/oder Decyl,
X für Chlorid oder Bromid, stehen.

Üblicherweise beträgt die Menge Phasentransferkatalysator, bevorzugt quartäres Ammoniumsalz der Formel (IV), von 0,001 bis 0,05 Mol, bevorzugt von 0,0015 bis 0,01 Mol, bezogen auf 1 Mol Alkylsulfid der Formel (II). Bevorzugt wird dabei der Phasentransferkatalysator, bevorzugt quartäres Ammoniumsalz der Formel (IV), dem Reaktionsgemisch hinzugefügt, bevor Base, bevorzugt Alkalimetallhydroxid, und Alkylsulfid der Formel (II) zum Reaktionsgemisch hinzugefügt werden. Darüber hinaus kann der Phasentransferkatalysator auch während oder vor der Umsetzung aus einfacheren Ausgangsstoffen hergestellt werden.

Das erfindungsgemäße Verfahren zur Herstellung von unsymmetrischen Dialkylsulfiden der Formel (I), umfassend die Umsetzung eines Alkylsulfides der Formel (II) mit einem Alkylhalogenid der Formel (III) wird bevorzugt bei Temperaturen von 15 bis 100 °C, bevorzugt von 15 bis 50 °C, weiterhin bevorzugt von 25 bis 40 °C, durchgeführt. Ist die Temperatur während der Reaktion zu niedrig, verlangsamt sich die Umsetzung und/oder erniedrigt sich die Ausbeute an Dialkylsulfid der Formel (I). Bei zu hoher Temperatur verringert sich die Löslichkeit des Alkylhalogenids der Formel (III) im Reaktionsgemisch, wodurch sich die Umsetzung, verlangsamt und/oder sich die Ausbeute an Dialkylsulfid der Formel (I) erniedrigt und/oder unerwünschte Nebenkomponenten entstehen können.

Während der Zugabe des Alkylhalogenids der Formel (III) zum Alkylsulfid der Formel (II), Phasentransferkatalysator, bevorzugt zum quartären Ammoniumsalz der Formel (IV), und ggf. Base, bevorzugt Alkalimetallhydroxid, steigt die Temperatur des Reaktionsgemisches durch exotherme Wärmeentwicklung an. Somit kann in der Regel auf eine kontinuierliche externe Wärmezuführung zum Reaktionsgemisch verzichtet werden.

In einer Ausführungsform kann die Temperatur des Reaktionsgemisches durch eine optionale externe Kühlung schneller in dem erforderlichen Temperaturbereich von 15 bis 100 °C, bevorzugt von 15 bis 50 °C, weiterhin bevorzugt von 25 bis 40 °C, gehalten werden. Dies ist dann erforderlich, wenn durch eine schnellere Zugabe von Base, bevorzugt Alkalimetallhydroxid, und Alkylsulfid der Formel (II), die Temperatur des Reaktionsgemisches stärker ansteigt als gewünscht. In einer alternativen Ausführungsform wird die Base, bevorzugt das Alkalimetallhydroxid, so zugegeben, dass der erforderliche Temperaturbereich ohne externe Kühlung eingehalten werden kann.

Die Alkylhalogenide der Formel (III), bevorzugt Chlormethan, Brommethan, Chlorethan, Bromethan, Chlorpropan, oder Brompropan, können dem Reaktionsgemisch in flüssiger oder gasförmiger Form zugefügt werden. Besonders bevorzugt ist das Alkylhalogenid der Formel (III) Chlormethan. Chlormethan siedet bei Umgebungsdruck bei -24 °C und wird daher bevorzugt gasförmig in das Reaktionsgemisch eingeleitet, besonders bevorzugt relativ nahe an der Flüssigkeitsoberfläche, beispielsweise im Bereich von 80 bis 95 % der Höhe des Reaktionsvolumens. Wenn das Reaktionsvolumen vom Boden des Reaktors bis zur Flüssigkeitsoberfläche, also der Grenzfläche zwischen der flüssigen und der gasförmigen Phase im Reaktor, beispielsweise eine Höhe von 1 Meter aufweist, wird das Alkylhalogenid der Formel (III) bevorzugt in einer Höhe 80 bis 95 cm, gerechnet vom tiefsten Punkte des Reaktorbodens, also 5 bis 20 cm unterhalb der Flüssigkeitsoberfläche eingeleitet. Bei dem erfindungsgemäßen Verfahren entsteht als Nebenprodukt das Salz der Base mit dem Rest X als Anion. Falls also Natriumhydroxid als Base und ein Alkylchlorid als Alkylhalogenid der Formel (III) in das erfindungsgemäße Verfahren eingesetzt werden, entsteht als Nebenprodukt Natriumchlorid. Aufgrund der schlechten Löslichkeit kann das entstehende Salz direkt in der Umgebung der Einleitungsstelle, beispielsweise des Einleitungsrohres ausfallen und gegebenenfalls die Öffnung verstopfen. Die optimale Dimensionierung und Positionierung der Einleitungsstelle, beispielsweise des Einleitungsrohres, kann der Fachmann abhängig von Form und Größe des verwendeten Reaktors durch einfache Versuche bestimmen. Wichtig ist hierbei nur, dass das Verstopfen der Einleitungsstelle, beispielsweise des Einleitungsrohres verhindert wird und der Masseneintrag des Alkylhalogenids der Formel (III) kontrollierbar ist.

Das Fortschreiten der Reaktion kann durch die Analyse von Proben aus dem Reaktionsgemisch festgestellt werden, die wie das Reaktionsgemisch aufgearbeitet worden sind. Der Gehalt an Edukt und Produkt kann dabei üblicherweise mittels HPLC oder Gaschromatografie, entweder als Flächenprozent ohne externem Standard oder als Gewichtsprozent mit externem Standard, ermittelt werden.

Nach beendeter Reaktion kann das Produkt, also das unsymmetrische Dialkylsulfid der Formel (I), vom Reaktionsgemisch abgetrennt werden, indem die hydraulische oder mechanische Durchmischung des Reaktionsgemischs beendet wird und gegebenenfalls der Druck innerhalb des Reaktors auf Umgebungsdruck angepasst wird. Dabei trennt sich das Reaktionsgemisch in eine erste obere organische und in eine zweite untere wässrige Phase auf. Die erste obere organische Phase enthält üblicherweise das unsymmetrische Dialkylsulfid der Formel (I) in einem Gehalt von 95 bis 99,9 Gew.%. Nach Abtrennung der zweiten unteren wässrige Phase kann die erste obere organische Phase dem Reaktor entnommen werden. Dabei wird das unsymmetrische Dialkylsulfid der Formel (I) in Ausbeuten von mindestens 95 Prozent der Theorie erhalten. Das so gewonnene unsymmetrische Dialkylsulfid der Formel (I) kann üblicherweise ohne weitere Aufarbeitung als Edukt für eine weitere Reaktion, beispielsweise in eine Epoxidierungsreaktionen, wie beispielsweise der Corey-Chaykovsky-Reaktion, einer Zwischenstufe von pharmazeutischen Wirkstoffen, beispielsweise Fluconazol, oder Pflanzenschutzwirkstoffen, beispielsweise Cyproconazol, eingesetzt werden. Falls das unsymmetrische Dialkylsulfid der Formel (I) in einer höheren Reinheit hergestellt werden muss, kann die Aufreinigung beispielsweise über Destillation erfolgen.

Überraschenderweise wurde hiermit ein einfaches, kostengünstiges und sicheres Verfahren bereitgestellt, mit dem erfindungsgemäß aus einem Alkylsulfid der Formel (II) und einem Alkylhalogenid der Formel (III) unsymmetrische Dialkylsulfide der Formel (I) in hohen Reinheiten und Ausbeuten erhalten werden und das nicht die Nachteile des Standes der Technik aufweist.

### Beispiele

### Beispiel 1: Butylmethylsulfid (erfindungsgemäß)

In einem Reaktor wurden 5500 g (59,76 Mol) Butanthiol bei 25 °C vorgelegt. Dazu wurden 7625 g Wasser und 60,1 g (0.13 Mol) Aliquat^{®} 336 (Mischung aus N-Methyl-N,N,N-trioctylammoniumchlorid und N-Methyl-N,N,N-tridecylammoniumchlorid,) gegeben, wodurch ein zweiphasiges Gemisch erzeugt wurde. Zu dem Gemisch wurden dann unter Vermischung zunächst 250 g wässrige Natronlauge (50 Gew.%, 3,14 Mol) gegeben, wobei die Temperatur zwischen 25 und 40 °C gehalten wurde. Nachfolgend wurden 3320 g (65,76 Mol) Chlormethan in dem geschlossenen Reaktor bei einem Anfangsdruck von 500 hPa unter die Flüssigkeitsoberfläche so zudosiert, dass der Druck im Reaktor nicht über 1200 hPa stieg. Parallel zu der Chlormethan-Zugabe wurden 4767 g wässrige Natronlauge (50 Gew.%, 59,58 Mol) innerhalb von 3 Stunden zudosiert, so dass die Temperatur des Reaktionsgemisches in dem Bereich von 25 bis 40 °C lag. Nach beendeter Zugabe von Chlormethan und Natronlauge wurde das Reaktionsgemisch weiter 30 Minuten bei 25 bis 40 °C vermischt. Nach Beendigung der Reaktion lag im Reaktor ein zweiphasiges Gemisch vor. Die untere wässrige Phase wurde zunächst aus dem Reaktor abgelassen und nachfolgend die obere Phase, die das Produkt enthält, dem Reaktor entnommen. Das Rohprodukt wurde so in einer Menge von 5995 g mit einer Reinheit von 99 Gew.% (56,95 Mol) erhalten, was einer Ausbeute von 95 % der Theorie entspricht.

## Patentansprüche

1. Verfahren zur Herstellung von unsymmetrischen Dialkylsulfiden der Formel (I),
R¹-S-R² (I),
umfassend die Umsetzung eines Alkylsulfides der Formel (II),
H-S-R² (II),
mit einem Alkylhalogenid der Formel (III),
R¹-X (III),
worin in den Formeln (I), (II) und/oder (III)
R¹ für einen C₁-C₃- Alkylrest, bevorzugt für Methyl,
R² für einen C₄-C₁₂ Alkylrest,
X für Halogen, bevorzugt für Chlorid, Bromid, Iodid, besonders bevorzugt für Chlorid, stehen,
zumindest in Gegenwart einer Base, bevorzugt in Gegenwart eines Alkalimetallhydroxides, wobei ein Reaktionsgemisch entsteht, und die Temperatur des Reaktionsgemisches während der Umsetzung in einem Bereich von 15 bis 100 °C, bevorzugt von 15 bis 50 °C, besonders bevorzugt von 25 bis 40 °C, liegt und
worin das Alkylhalogenid der Formel (III) in einer Menge von 0,9 bis 3 Moläquivalenten bezogen auf das Alkylsulfid der Formel (II) eingesetzt wird und
wobei die Umsetzung des Alkylhalogenids der Formel (III) und des Alkylsulfides der Formel (II) in Abwesenheit von organischen Lösungsmitteln erfolgt.

2. Verfahren nach Anspruch 1, wobei die Umsetzung in Gegenwart von Wasser und einem Phasentransferkatalysator, bevorzugt einem quartären Ammoniumsalz der Formel (IV),
R³R⁴₃N⁺Y⁻ (IV),
worin
R³ für Wasserstoff oder für Methyl, bevorzugt für Methyl,
R⁴ für einen C₄-C₁₂ Alkylrest, bevorzugt für Octyl und/oder Decyl,
X für Halogen, bevorzugt für Chlorid, Bromid, oder Iodid, stehen,
erfolgt.

3. Verfahren nach Anspruch 1 oder 2, wobei R² in Formel (I) und Formel (II) für n-Butyl, n-Pentyl, n-Hexyl, n-Heptyl, n-Octyl, n-Nonyl, n-Decyl, n-Undecyl or n-Dodecyl steht.

4. Verfahren nach einem der Ansprüche 1 bis 3, worin das Alkylhalogenid der Formel (III) in einer Menge von 1,1 bis 1,5 Moläquivalenten bezogen auf das Alkylsulfid der Formel (II) eingesetzt wird.

5. Verfahren nach einem der Ansprüche 2 oder 3, worin der Phasentransferkatalysator, bevorzugt das quartäre Ammoniumsalz der Formel (IV) in einer Menge von 0,001 bis 0,05 Mol, bevorzugt von 0,0015 bis 0,01 Mol, bezogen auf 1 Mol Alkylsulfid der Formel (II) eingesetzt wird.

6. Verfahren nach einem der Ansprüche 1 bis 5, worin die Base in einer Menge von 0,9 bis 2 Moläquivalenten, bevorzugt von 1,0 bis 1,2 Moläquivalenten, bezogen auf das Alkylsulfid der Formel (II) eingesetzt wird.

7. Verfahren nach einem der Ansprüche 1 bis 6, worin die Base, bevorzugt das Alkalimetallhydroxid, Natriumhydroxid oder Kaliumhydroxid ist.

8. Verfahren nach einem der Ansprüche 2 bis 7, worin zu einer Mischung des Alkylsulfides der Formel (II) und dem Phasentransferkatalysator, bevorzugt dem quartären Ammoniumsalz der Formel (IV), und gegebenenfalls Wasser, gleichzeitig Base, bevorzugt das Alkalimetallhydroxid, und Alkylhalogenid der Formel (III), gegeben wird.

9. Verfahren nach einem der Ansprüche 2 bis 8, wobei die Umsetzung des Alkylhalogenids der Formel (III) und des Alkylsulfides der Formel (II) in Gegenwart von Base, bevorzugt des Alkalimetallhydroxids, derart erfolgt, dass zu Beginn der Umsetzung zu der Mischung zumindest enthaltend Alkylsulfid der Formel (II), gegebenenfalls Phasentransferkatalysator, bevorzugt quartäres Ammoniumsalz der Formel (IV), und gegebenenfalls Wasser, von 0,01 bis 0,05 Mol, bevorzugt 0,02 bis 0,04 Mol, Base, bevorzugt Alkalimetallhydroxid, bezogen auf 1,0 Mol Alkylsulfid der Formel (II) zugegeben wird, bevor Alkylhalogenid der Formel (III) zum Reaktionsgemisch zugegeben wird.

10. Verfahren nach einem der Ansprüche 1 bis 9, wobei die Umsetzung in einem geschlossenen Reaktor erfolgt.

11. Verfahren nach Anspruch 10, wobei der Partialdruck an Alkylhalogenid der Formel (III) in dem gasförmigen Raum oberhalb des flüssigen Reaktionsgemisches innerhalb des geschlossenen Reaktors von 100 bis 1500 hPa, bevorzugt von 100 bis 700 hPa, beträgt.

## Claims

1. Process for preparing unsymmetrical dialkyl sulfides of formula (I),
R¹-S-R² (I),
comprising the reaction of an alkyl sulfide of formula (II),
H-S-R² (II),
with an alkyl halide of formula (III),
R¹-X (III),
wherein in formulae (I), (II) and/or (III)
R¹ is a C₁-C₃ alkyl radical, preferably methyl,
R² is a C₄-C₁₂ alkyl radical,
X is halogen, preferably chloride, bromide, iodide, particularly preferably chloride,
at least in the presence of a base, preferably in the presence of an alkali metal hydroxide, wherein a reaction mixture is formed, and the temperature of the reaction mixture during the reaction is in a range from 15°C to 100°C, preferably from 15°C to 50°C, particularly preferably from 25°C to 40°C, and
wherein the alkyl halide of formula (III) is used in an amount of 0.9 to 3 molar equivalents based on the alkyl sulfide of formula (II) and
wherein the reaction of the alkyl halide of formula (III) and of the alkyl sulfide of formula (II) is performed in the absence of organic solvents.

2. Process according to Claim 1, wherein the reaction is performed in the presence of water and a phase transfer catalyst, preferably a quaternary ammonium salt of formula (IV),
R³R⁴₃N⁺Y⁻ (IV),
in which
R³ is hydrogen or methyl, preferably methyl,
R⁴ is a C₄-C₁₂ alkyl radical, preferably octyl and/or decyl,
X is halogen, preferably chloride, bromide or iodide.

3. Process according to Claim 1 or 2, wherein R² in formula (I) and formula (II) is n-butyl, n-pentyl, n-hexyl, n-heptyl, n-octyl, n-nonyl, n-decyl, n-undecyl or n-dodecyl.

4. Process according to any of Claims 1 to 3, wherein the alkyl halide of formula (III) is used in an amount of 1.1 to 1.5 molar equivalents based on the alkyl sulfide of formula (II).

5. Process according to either of Claims 2 and 3, wherein the phase transfer catalyst, preferably the quaternary ammonium salt of formula (IV), is used in an amount of 0.001 to 0.05 mol, preferably of 0.0015 to 0.01 mol, based on 1 mol of alkyl sulfide of formula (II).

6. Process according to any of Claims 1 to 5, wherein the base is used in an amount of 0.9 to 2 molar equivalents, preferably of 1.0 to 1.2 molar equivalents, based on the alkyl sulfide of formula (II).

7. Process according to any of Claims 1 to 6, wherein the base, preferably the alkali metal hydroxide, is sodium hydroxide or potassium hydroxide.

8. Process according to any of Claims 2 to 7, wherein base, preferably the alkali metal hydroxide, and alkyl halide of formula (III) are simultaneously added to a mixture of the alkyl sulfide of formula (II) and the phase transfer catalyst, preferably the quaternary ammonium salt of formula (IV), and optionally water.

9. Process according to any of Claims 2 to 8, wherein the reaction of the alkyl halide of formula (III) and of the alkyl sulfide of formula (II) in the presence of base, preferably the alkali metal hydroxide, is performed in such a way that at the beginning of the reaction from 0.01 to 0.05 mol, preferably 0.02 to 0.04 mol, of base, preferably alkali metal hydroxide, based on 1.0 mol of alkyl sulfide of formula (II) is added to the mixture at least comprising alkyl sulfide of formula (II), optionally phase transfer catalyst, preferably quaternary ammonium salt of formula (IV), and optionally water, before alkyl halide of formula (III) is added to the reaction mixture.

10. Process according to any of Claims 1 to 9, wherein the reaction is performed in a closed reactor.

11. Process according to Claim 10, wherein the partial pressure of alkyl halide of formula (III) in the gaseous space above the liquid reaction mixture within the closed reactor is from 100 to 1500 hPa, preferably from 100 to 700 hPa.

## Revendications

1. Procédé de préparation de sulfures de dialkyle asymétriques de formule (I),
R¹-S-R² (I),
comprenant la transformation d'un sulfure d'alkyle de formule (II),
H-S-R² (II),
avec un halogénure d'alkyle de formule (III),
R¹-X (III),
où, dans les formules (I), (II) et/ou (III),
R¹ représente un radical C₁-C₃-alkyle, de préférence méthyle,
R² représente un radical C₄-C₁₂-alkyle,
X représente halogène, de préférence chlorure, bromure, iodure, de manière particulièrement préférée chlorure,
au moins en présence d'une base, de préférence en présence d'un hydroxyde de métal alcalin, un mélange réactionnel se formant et la température du mélange réactionnel pendant la transformation se situant dans une plage de 15 à 100°C, de préférence de 15 à 50°C, de manière particulièrement préférée de 25 à 40°C et
l'halogénure d'alkyle de formule (III) étant utilisé en une quantité de 0,9 à 3 équivalents molaires par rapport au sulfure d'alkyle de la formule (II) et
la transformation de l'halogénure d'alkyle de formule (III) et du sulfure d'alkyle de formule (II) ayant lieu en l'absence de solvants organiques.

2. Procédé selon la revendication 1, la transformation ayant lieu en présence d'eau et d'un catalyseur de transfert de phase, de préférence d'un sel d'ammonium quaternaire de formule (IV),
R³R⁴₃N⁺Y⁻ (IV),
dans laquelle
R³ représente hydrogène ou méthyle, de préférence méthyle,
R⁴ représente un radical C₄-C₁₂-alkyle, de préférence octyle et/ou décyle,
X représente halogène, de préférence chlorure, bromure ou iodure.

3. Procédé selon la revendication 1 ou 2, R² dans la formule (I) et dans la formule (II) représentant n-butyle, n-pentyle, n-hexyle, n-heptyle, n-octyle, n-nonyle, n-décyle, n-undécyle ou n-dodécyle.

4. Procédé selon l'une des revendications 1 à 3, l'halogénure d'alkyle de formule (III) étant utilisé en une quantité de 1,1 à 1,5 équivalent molaire par rapport au sulfure d'alkyle de formule (II).

5. Procédé selon l'une des revendications 2 ou 3, le catalyseur de transfert de phase, de préférence le sel d'ammonium quaternaire de formule (IV), étant utilisé en une quantité de 0,001 à 0,05 mole, de préférence de 0,0015 à 0,01 mole, par rapport à 1 mole de sulfure d'alkyle de formule (II).

6. Procédé selon l'une des revendications 1 à 5, la base étant utilisée en une quantité de 0,9 à 2 équivalents molaires, de préférence de 1,0 à 1,2 équivalent molaire, par rapport au sulfure d'alkyle de la formule (II).

7. Procédé selon l'une des revendications 1 à 6, la base, de préférence l'hydroxyde de métal alcalin, étant l'hydroxyde de sodium ou l'hydroxyde de potassium.

8. Procédé selon l'une des revendications 2 à 7, dans lequel on ajoute, à un mélange du sulfure d'alkyle de formule (II) et du catalyseur de transfert de phase, de préférence du sel d'ammonium quaternaire de formule (IV), et le cas échéant d'eau, simultanément la base, de préférence l'hydroxyde de métal alcalin, et l'halogénure d'alkyle de formule (III).

9. Procédé selon l'une des revendications 2 à 8, la transformation de l'halogénure d'alkyle de formule (III) et du sulfure d'alkyle de formule (II) en présence d'une base, de préférence de l'hydroxyde de métal alcalin, ayant lieu de telle sorte qu'au début de la transformation, on ajoute au mélange, contenant au moins le sulfure d'alkyle de formule (II), le cas échéant le catalyseur de transfert de phase, de préférence le sel d'ammonium quaternaire de formule (IV), et le cas échéant de l'eau, 0,01 à 0,05 mole, de préférence 0,02 à 0,04 mole, de base, de préférence d'hydroxyde de métal alcalin, par rapport à 1,0 mole de sulfure d'alkyle de formule (II) avant l'ajout de l'halogénure d'alkyle de formule (III) au mélange réactionnel.

10. Procédé selon l'une des revendications 1 à 9, la transformation ayant lieu dans un réacteur fermé.

11. Procédé selon la revendication 10, la pression partielle d'halogénure d'alkyle de formule (III) dans l'espace gazeux au-dessus du mélange réactionnel liquide dans le réacteur fermé représentant 100 à 1500 hPa, de préférence 100 à 700 hPa.
